(19)

Europäisches
Patentamt
European
Patent Office
Office européen
des brevets

(11)  **EP 2 493 487 B1**

(12)  **EUROPEAN PATENT SPECIFICATION**

(45) Date of publication and mention
of the grant of the patent:
**24.08.2016 Bulletin 2016/34**

(21) Application number: **10768979.6**

(22) Date of filing: **27.10.2010**

(51) Int Cl.:
*A61K 35/18* [(2006.01)]        *A61K 38/43* [(2006.01)]
*A61P 3/00* [(2006.01)]

(86) International application number:
**PCT/EP2010/066269**

(87) International publication number:
**WO 2011/051346 (05.05.2011 Gazette 2011/18)**

(54) **COMPOSITION TO INDUCE SPECIFIC IMMUNE TOLERANCE**

ZUSAMMENSETZUNG ZUR INDUZIERUNG EINER SPEZIFISCHEN IMMUNTOLERANZ

COMPOSITION INDUISANT UNE TOLÉRANCE IMMUNITAIRE SPÉCIFIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.10.2009 US 255250 P
19.04.2010 US 325511 P**

(43) Date of publication of application:
**05.09.2012 Bulletin 2012/36**

(73) Proprietor: **Erytech Pharma
69008 Lyon (FR)**

(72) Inventors:
• **GODFRIN, Yann
F-69004 Lyon (FR)**
• **BANZ, Alice
F-69007 Lyon (FR)**

(74) Representative: **Lavoix
62, rue de Bonnel
69448 Lyon Cedex 03 (FR)**

(56) References cited:
**WO-A1-96/13517        WO-A1-2009/019317
WO-A2-2006/016247      US-A1- 2003 045 467**

• **SERAFINI S ET AL: "Drug delivery through
phagocytosis of red blood cells", TRANSFUSION
MEDICINE AND HEMOTHERAPY, KARGER,
BASEL, CH, vol. 31, no. 2, 1 March 2004
(2004-03-01), pages 92-101, XP008117339, ISSN:
1660-3796, DOI: DOI:10.1159/000078042**
• **BUSTOS N L ET AL: "Enzyme replacement
therapy in porphyrias--V. In vivo correction of
delta-aminolaevulinate dehydratase defective in
erythrocytes in lead intoxicated animals by
enzyme-loaded red blood cell ghosts", DRUG
DESIGN AND DELIVERY, HARWOOD ACADEMIC
PUBLISHERS GMBH, XX, vol. 5, no. 2, 1 December
1989 (1989-12-01), pages 125-131, XP009143482,**
• **HAMIDI ET AL: "Applications of carrier
erythrocytes in delivery of biopharmaceuticals",
JOURNAL OF CONTROLLED RELEASE,
ELSEVIER, AMSTERDAM, NL, vol. 118, no. 2, 2
March 2007 (2007-03-02) , pages 145-160,
XP005912142, ISSN: 0168-3659, DOI:
DOI:10.1016/J.JCONREL.2006.06.032**
• **MILLAN C G ET AL: "Drug, enzyme and peptide
delivery using erythrocytes as carriers",
JOURNAL OF CONTROLLED RELEASE,
ELSEVIER, AMSTERDAM, NL, vol. 95, no. 1, 20
February 2004 (2004-02-20), pages 27-49,
XP004487812, ISSN: 0168-3659, DOI:
DOI:10.1016/J.JCONREL.2003.11.018**

Remarks:
The file contains technical information submitted after
the application was filed and not included in this
specification

**Description**

The present invention relates to a composition which induces, in a host, an immune tolerance to a peptidic or proteic active principle, in particular a therapeutic peptide, polypeptide or protein, a peptidic or proteic autoantigen, a peptide, polypeptide or protein inducing an allergic reaction or a transplantation peptidic or proteic antigen. The invention also relates to a method of treatment of a mammal, including human.

The liver is known to favour the induction of immune tolerance. This is exemplified by tolerization of food antigens in the liver and acceptance of liver allografts. There has been also some demonstration of antigen-specfic tolerance to some foreign antigens delivered into the liver. E. Breous et al., Hepatology, August 2009, pp612-621 report that hepatic regulatory T cells and Kupffer cells are crucial mediators of systemic T cell tolerance to antigens targeting murine liver. They report that in a model of liver-directed gene transfer, cytotoxic T lymphocyte responses to non-self antigens are controlled by hepatic regulatory T cells that secrete the immunosuppressive cytokine interleukin IL-10 in response to the antigen. In addition, the Kupffer cells are rendered tolerogenic rather than generating an immune response in this context.

The tolerogenic role of Kupffer cells has also been reported by C. Ju et al., Chem. Res. Toxicol. 2003, 16:1514-1519. See also A.H. Lau et al., Gut 2003, 52:1075-1078.

The present invention aims at providing compositions that can be used for the induction of an immune tolerance against a variety of peptidic or proteic active principles. It aims in particular at providing a specific immune tolerance with respect to one or several peptidic or proteic active principles.

An object of the invention is therefore a composition according to claim 1, for use to inducing, in a host, an immune tolerance to a peptidic or proteic active principle.

The active principle may be of natural, synthetic or recombinant origin. By "containing" molecule, it is intended to encompass molecules that contain the peptide, polypeptide or protein of interest and another moiety that may be of any origin and is not detrimental to the action of said peptide, polypeptide or protein. For example, such moiety includes haptens.

Without being bound to theory, the composition according to the invention is deemed to induce antigen(s)-specific regulatory T cells (Tregs) and to produce immunosuppressive cytokines or interleukins, in particular IL-10.

Biological and/or biotechnology-derived peptides, polypeptides or proteins are increasingly used as therapeutic agents. It has been however recognised that these agents may induce humoral and/or cellular immune responses. The consequences of an immune reaction to such therapeutic agent range from transient appearance of antibodies without any clinical significance to severe life threatening conditions. Potential clinical consequences are severe hypersensitivity-type reactions, decrease in efficacy and induction of auto-immunity, including antibodies to the endogenous form of the peptide, polypeptide or protein (European Medicines Agency, Committee for Medicinal products for human use (CHMP), Guidelines on immunogenicity assessment of biotechnology-derived therapeutic proteins, Draft, London, 24 January 2007).

A therapeutic peptide, polypeptide or protein is by definition a peptide, polypeptide or protein or a peptide, polypeptide or protein containing molecule that is efficient in treating a pathology, especially a pathology due to a deficiency that can be corrected by administration of this molecule.

In an embodiment, the therapeutic peptide, polypeptide or protein is an antibody. This encompasses any fragment thereof.

In another embodiment, the therapeutic peptide, polypeptide or protein is a clotting factor. This encompasses any fragment thereof.

In another embodiment, the therapeutic peptide, polypeptide or protein is an enzyme. This encompasses any fragment thereof.

In another embodiment, the therapeutic peptide, polypeptide or protein is a growth factor. This encompasses any fragment thereof.

The term fragment is used to encompass any fragment of the peptide, polypeptide or protein that is known to be efficient in treating the associated pathology in replacement to the whole molecule.

The glycosylated forms are also encompassed by these definition.

In an embodiment, the active principle is a lysosomal enzyme. The lysosomal enzyme may be one used to treat or correct a lysosomal storage disease by enzyme replacement therapy (ERT), including pompe disease (Glycogen storage disease type II), Fabry disease and Mucopolysaccharidoses disorders MPS I. As examples, one may mention:

- alphaglucosidase enzyme, e.g. Myozyme® to treat pompe disease;
- laronidase, e.g. Aldurazyme® to treat MPS I;
- alphagalactosidase A or agalsidase Alpha, e.g. Fabrazyme® and Replagal® to treat the Fabry disease.

In another embodiment, the active principle is a clotting factor useful in treating Haemophilia. The clotting factor may be Factor VIII, in particular for treating Haemophilia A. The clotting factor may be Factor IX, in particular for treating Haemophilia B. The clotting factor may be Factor VII for treating both Haemophilias.

A peptidic or proteic autoantigen is by definition an antigen that is a normal tissue constituent and is in a patient the target of a detrimental humoral or cell-mediated immune response, as in autoimmune disease.

In an embodiment, the active principle is against Rheumatoid Arthritis (RA).

In another embodiment, the active principle is against Multiple Sclerosis (MS). For example the active principle is myelin basic protein.

In another embodiment, the active principle is against Juvenile diabete, such as diabetes type 1 and LADA (Latent Autoimmune Diabetes of Adults). As examples, one may cite the Beta-cell antigen, in particular glutamic acid decarboxylase (GAD), the pro-insuline and the insuline-like growth factor-2 (IGF2), and mixtures thereof.

In another embodiment, the active principle is against Uveitis. One may cite the retinal-S antigen.

In another embodiment, the active principle is against an inflammatory bowel disease (IBD), such as Crohn's disease and ulcerative colitis.

In another embodiment, the active principle is against systemic lupus erythematosus.

In another embodiment, the active principle is against psoriasis.

In another embodiment, the active principle is against acquired myasthenia gravis. As example, one may cite the acetyl choline receptor.

A peptide, polypeptide or protein inducing an allergic reaction is by definition a peptide, polypeptide or protein which is responsible for an allergic reaction in a host which reaction may include anaphylactic chock.

In an embodiment, this peptide, polypeptide or protein inducing an allergic reaction is a therapeutic active peptide, polypeptide or protein as mentioned above, wherein the present invention allows avoiding some or any allergic reaction against it and neutralization thereof.

In another embodiment, the peptide, polypeptide or protein inducing an allergic reaction is of food origin or any other proteic for peptidic molecule that may enter the blood circulation and create allergic reaction, e.g. after oral ingestion.

A transplantation peptidic or proteic antigen is by definition an antigen that is presented by the transplanted tissue and is involved in the patient in the graft rejection, say Graft Versus Host Disease (GVHD).

In an embodiment, the transplantation antigen is one involved in kidney graft rejection.

In an embodiment, the transplantation antigen is one involved in heart graft rejection.

In an embodiment, the transplantation antigen is one involved in liver graft rejection.

The term "host" refers preferably to humans, but also to animals, in particular pets (especially dogs or cats) and animals for sport (especially horses).

According to the invention, the red blood cells contain, i.e. encapsulate, the active principle (AP), which means that the AP is or is essentially inside the red blood cells.

In an instance, the composition targets the antigen-presenting cells (APCs) of the reticuloendothelial system. According to a feature, the red blood cells are designed, selected or modified so as to promote targeting of the antigen-presenting cells (APCs) of the reticuloendothelial system.

In a preferred instance, the composition targets the liver and especially the Kupffer cells. According to a feature, the red blood cells are designed, selected or modified so as to promote targeting of the liver. Delivering the AP to the liver results in the induction of AP tolerance and especially AP-specific tolerance. The liver's tolerogenic APCs are implicated in the induction of this tolerance. These cells are essentially Kupffer cells (KCs), non mature hepatic dendritic cells and liver sinusoidal endothelial cells.

In a preferred instance, the composition is used to repress the proinflammatory response of APCs. According to a feature, the red blood cells are preferably designed or modified so as to repress the proinflammatory response of APCs.

In a preferred instance, the compositions according to the invention comprise red blood cells which contain the AP and target the liver. The composition promotes phagocytosis of these red blood cells by the liver's APCs, especially the KCs.

According to a first instance, the red blood cells contain the AP and are in the form of an immune complex with an immunoglobulin which recognizes an epitope at the surface of said red blood cells, so as to promote the phagocytosis of said red blood cells by the liver's APCs, especially the KC.

The composition also makes it possible to promote phagocytosis by macrophages.

Preferably, the immunoglobulin is an immunoglobulin G.

As antibody that may be used to make adequate opsonisation, one may mention anti-Rhesus antibodies, anti-glycophorine A antibodies and anti-CR1 (CR1 = type 1 complement receptor) antibodies. Anti-Rhesus antibodies are preferred.

According to another instance, the liver targeting and/or the inhibition of the proinflammatory response is/are done by an, appropriate chemical treatment using agents which modify the surface of red blood cells, and in particular bridging or crosslinking agents such as bis(sulphosuccinimidyl) suberate (BS3 or $BS^3$), glutaraldehyde or neuraminidase.

According to another instance, the liver targeting and/or the inhibition of the proinflammatory response is/are done by using a ionophore. By ionophore, it is meant as it is well known from the person skilled in the art a lipid-soluble molecule that allows the transport of ions across the lipid bilayer of the cell membrane. Ionophores may be in particular lipid-soluble molecules as synthesized by microorganisms to transport ions across the lipid bilayer of the cell membrane. Generally, the ionophore is able to form a complex with a ion and serves as ion-carrier.

In an instance, the ionophore is one forming a complex with a divalent cation such as calcium. According to the invention, the ionophore may be used with calcium, which induces an increase of the calcium intracellular concentration and an exposition of the phosphatidylserine, leading to an early aging of the red blood cells.

As an example, one may use the calcium ionophore A23187 (calcimycin). It is deemed that the ionophore such as A23187 induces a raise in intracellular calcium concentrations of the RBCs, leading to the senescence of the cells and that the phagocytosis of aged red blood cells represses the proinflammatory response. This is in accordance with Romero P.J., Romero E.A., Blood Cells Mol. Dis. 25 (1999) 9-19; and Bratosin D. et al., Cell Death Differ. 8 (2001) 1143-1156.

In a particular instance, at least two methods of targeting are combined, and, for example, the composition then comprises AP-containing red blood cells which are in the form of an immune complex and are chemically treated so as to promote their uptake in the liver, and phagocytosis by APCs, in particular by the KCs.

In an instance the red blood cells originate from the patient itself.

In another instance, the red blood cells originate from a blood-typing compatible donor.

The composition according to the invention may comprise one or more APs in the same red blood cells or each one in different red blood cells.

Techniques for encapsulating active ingredients in red blood cells are known and the basic technique by lysis-resealing, is described in patents EP-A-101 341 and EP-A-679 101. According to this technique, the primary compartment of a dialysis element (for example, a dialysis tubing or a dialysis cartridge) is continuously fed with a suspension of red blood cells, while the secondary compartment contains an aqueous solution which is hypotonic with respect to the suspension of red blood cells, in order to lyse the red blood cells; next, in at resealing unit, the resealing of the red blood cells is induced in the presence of the AP by increasing the osmotic and/or oncotic pressure, and then a suspension of red blood cells containing the AP is collected.

Among the variants described up until now, preference is given to the method described in WO2006/016247, which makes it possible to efficiently, reproducibly, safely and stably encapsulate the AP. This method comprises the following steps:

1 - suspension of a red blood cell pellet in an isotonic solution at a haematacrit level greater than or equal to 65%, cooling between + 1 and + 8°C,
2 - measurement of the osmotic fragility using a sample of red blood cells from said red blood cell pellet, it being possible for steps 1 and 2 to be carried out in any order (including in parallel),
3 - lysis and internalization process of the AP, inside the same chamber, at a temperature constantly maintained between + 1 and + 8°C, comprising passing the suspension of red blood cells at a haematocrit level greater than or equal to 65%, and a hypotonic lysis solution cooled to between + 1 and 8°C, through a dialysis cartridge; and the lysis parameters being adjusted according to the osmotic fragility previously measured; and
4 - resealing process carried out in a second chamber, inside which the temperature is between + 30 and + 40°C, and in the presence of a hypertonic solution.

The "internalization" is intended to mean penetration of the AP inside the red blood cells.

In particular, for the dialysis, the red blood cell pellet is suspended in an isotonic solution at a high haematocrit level, greater than or equal to 65%, and preferably greater than or equal to 70%, and this suspension is cooled to between + 1 and + 8°C, preferably between + 2 and 6°C, typically in the region of + 4°C. According to a specific embodiment, the haematocrit level is between 65% and 80%, preferably between 70% and 80%.

The osmotic fragility is advantageously measured on the red blood cells just before the lysis step. The red blood cells or the suspension containing them are (is) advantageously at a temperature close to or identical to the temperature selected for the lysis. According to another advantageous feature of the invention, the osmotic fragility measurement is exploited rapidly, i.e. the lysis process is carried out shortly after the sample has been taken. Preferably, this period of time between taking the sample and beginning the lysis is less than or equal to 30 minutes, more preferably still less than or equal to 25, and even less than or equal to 20 minutes.

As regards the manner in which the lysis-resealing process is carried out, with the osmotic fragility being measured and taken into account, is discribed in WO2006/016247.

According to one feature of the invention, the composition according to the invention comprises, at the end, a suspension of red blood cells at a haematocrit level of between about 40% and about 70%, preferably between about 45% and about 55%, better still about 50%. It is preferably packaged in a volume of about 1 to about 250 ml. The packaging is preferably in a blood bag, syringe and the like, of a type suitable for blood transfusion or administration. The amount of encapsulated AP corresponding to the medical prescription is preferably entirely contained in the blood bag, syringe and the like.

The invention also discloses a method for inducing, in a host, an immune tolerance to a peptidic or proteic active principle, said composition comprising red blood cells containing an active principle selected from the group consisting of a therapeutic peptide, polypeptide or protein, a peptidic or proteic autoantigen, a peptide, polypeptide or protein inducing an allergic reaction and a transplantation peptidic or proteic antigen. This method comprises the administration to the host of an effective amount of a composition according to the invention, in particular Intravenously, by injection or infusion, preferably by infusion.

According to this disclosure, about 1 to about 250 ml, especially about 10 to about 250 ml, typically about 10 and about 200 ml of a suspension of red blood cells is administered. The suspension is at an appropriate haematocrit level, generally of between about 40% and about 70%, preferably between about 45% and about 55%, better stili about 50%, are administered. The red blood cells may have their own tolerogenic effect with respect to the active principle that is presented at the same time (the encapsulated active principle). High amounts of red blood cells may thus favour the tolerogenic effect. On the other hand, targeting the liver as recited above may allow to use low doses of red blood cells. The person skilled in the art may thus select the optimal amount of active principle and of red blood cells used in a patient, and may take into account whether or not the red blood cells have been treated to target the liver.

An object of the invention is also the use of a composition according to the invention, for the induction of an immune tolerance specific to the active principle or the active principles that are present in the administered red blood cells.

Another object of the invention is a composition according to the invention, for use as a medicament to induce an immune tolerance specific to the active principle or the active principles that are present in the administered red blood cells.

The present invention will now be described in greater detail by means of embodiments taken by way of nonlimiting examples, and which refer to the attached drawings wherein:

Figure 1 is a graph representing the percentage of CD4 T cells expressing FOXP3
Figure 2 is a graph representing the percentage of regulatory CD4$^+$ CD25$^+$ T cells producing IL-10.
Figure 3 is a graph representing the percentage of CD4 T cells expressing FOXP3 in the spleen.
Figure 4 is a graph representing the percentage of CD4 T cells expressing FOX P3 in the liver.
Figure 5 is a graph representing the percentage of OVA-specific CD8 T cells.

Example 1 : Encapsulation of FITC-dextran in murine red blood cells

FITC-dextran fluorochrome (70 kDa) has been encapsulated in red blood cells of murine origin (OF1 mice) using the column hypotonic dialysis. Blood is centrifuged and then washed 3 times with PBS. Heamatocrit is adjusted to 70 % in the presence of FITC-dextran added to a final concentration of 8 mg/ml before dialysis. The red blood cells are dialysed at a rate of 2 ml/min against a lysis tampon having a low osmolarity (counter-flux at 15 ml/min). The lysed red blood cells leaving the column are rescelled using a high osmolarity solution and incubation 30 min at 37 °C. After several washings with PBS containing glucose, the cells are brought to heamatocrit 50 %.

Example 2. Chemical treatment with bis(sulphosuccinimidyl) suberate (BS3) 1 mM on the red blood cells containing FITC-dextran

The suspension of red blood cells encapsulating FITC-dextran is washed several times before being brought to 1.7 x 10$^6$ cell/$\mu$l with PBS and mixed with one volume of a buffer solution of 2 mM BS3 (the BS3 solution contains glucose 0.09 % and phosphate buffer, pH 7.4), so as to obtain a final BS3 concentration of 1 mM. The cells are incubated for 30 minutes at room temperature. The reaction is quenched by adding one volume of 20 mM Tris-HCl, NeCl 140 mM. After incubation at room temperature for 5 minutes, the mixture is centrifuged at 800 g for 5 min, 4°C. The cells are then washed twice with PBS containing glucose (centrifugation at 800 g) and once with SAG-BSA 6 % (centrifugation at 1000 g) for 10 min, before adjustement to heamatocrit 50 % to constitute the final products.

Example 3. Chemical treatment with bis(sulphosuccinimidyl) suberate (BS3) 5 mM on the red blood cells containing FITC-dextran

The suspension of red blood cells encapsulating FITC-dextran is washed several times before being brought to 1.7 x 10$^6$ call/$\mu$l with PBS and mixed with one volume of a buffer solution of 10 mM BS3 (the BS3 solution contains glucose 0.09 % and phosphate buffer, pH 7.4), so as to obtain a final BS3 concentration of 5 mM. The cells are incubated for 30 minutes at room temperature. The reaction is quenched by adding one volume of 20 mM Tris-HCl, NaCl 140 mM.

After incubation at room temperature for 5 minutes, the mixture is centrifuged at 800 g for 5 min. 4°C. The cells are then washed twice with PBS containing glucose (centrifugation at 800 g) and once with SAG-BSA 6 % (centrifugation at 1000 g) for 10 min, before adjustement to heamatocrit 50 % to constitute the final products.

Example 4. Treatment of the red blood cells containing FITC-dextran by the A23187 ionophore

The suspension of red blood cells containing FITC-dextran is washed once with a tampon A containing Hepes 10 mM, NaCl 140 mM, BSA 0.1%, CaCl$_2$ 2.5 mM, and then the suspension is diluted to 1.10$^6$ cells/microliter using tampon A. Ionophore concentrated in DMSO is diluted with tampon A and then added to the cell suspension in order to get a final concentration of 0.15, 0.2 or 0.3 $\mu$M. The cells are incubated 30 min at 37 °C. The mixture is centrifuged at 800 g during 6 min, 4 °C. Then the cells are washed 2 times with PBS containing glucose (centrifugation 800 g) and once with SAG-BSA 6% (centrifugation 1000 g), and the final products are obtained.

Example 5. Biodistribution of FITC-dextran after injection of the red blood cells in mice

5 batches of red blood cells from mice OF1 containing FITC-dextran as obtained in example 1 and treated or not with BS3 or ionophore (based on examples 2-4) as follows were prepared:

Batch 1: no treatment
Batch 2: BS3 1 mM
Batch 3: BS3 5 mM
Batch 4: ionophore 0.2 $\mu$M
Batch 5: ionophore 0.3 $\mu$M.

Each batch is injected IV at J.1 into OF1 mice. The mice are sacrificed 1h30 after injection, and blood, spleen, liver and bone marrow are recovered: aliquots of 50 $\mu$l of blood and for spleen, liver and bone marrow aliquots of 50 $\mu$l after grinding and homogeneisation of the whole cells of each organ. The aliquots are congelated for at least 20 min at -20 °C, then thawn slowly at room temperature. The aliquots from the control mice are used to prepare a FITC-dextran standard range of concentration: the aliquots are then lysed with 125 $\mu$l of different concentrations of FITC-dextran to constitute the standard range of concentration.

The aliquots of the sample to be analysed are lysed using 125 $\mu$l of distilled water. Then 175 $\mu$l of TCA 12 % are added to the aliquots. The mixtures are then centrifuged at 15,000 g, 10 min, 4 °C. 200 $\mu$l of acid supernatant are taken and 500 $\mu$l of triethanolamine 0.4 M are added before fluorimetry detection (excitation at 494 nm, emission 521 nm). The FITC-dextran concentration of each sample can be determined using the standard range of concentration and the proportion of FITC-dextran present in the corresponding organ can then be deduced.

Biodistribution of FITC-dextran 1h30 after injection of the red blood calls in OF1 mice (Table 1):

| Batches | Blood | Liver | Spleen |
|---|---|---|---|
| 1 | 57% | 19.8 $\pm$ 7,6% | 7.7 $\pm$ 1,9 % |
| 2 | 35.5 $\pm$ 8% | 32 $\pm$ 4,6% | 22.8 $\pm$ 14 % |
| 3 | 11% | 20,6% | 13.30% |
| 4 | 19.9 $\pm$ 12,6% | 24 $\pm$ 1,3% | 7.2 $\pm$ 1,2% |
| 5 | 14.7 $\pm$ 1,3% | 36.7 $\pm$ 7,2 % | 9.2 $\pm$ 0,8 % |

1 mM BS3 treatment indices erythrocyrte targeting of the liver and the spleen whereas ionophore treatment induces erythrocyrte targeting of the liver only. Increasing dose of ionophore enhances targeting.

Example 6. Phagocytosis measurement in mice of FITC-dextran containing red blood cells

5 batches of red blood cells from mice OF1 containing FITC-dextran as obtained in example 1 and treated or not with BS3 or ionophore (based on examples 2-4) were prepared:

Batch 1: no treatment
Batch 2: BS3 1 mM
Batch 3: BS3 5 mM

Batch 4: ionophore 0.2 µM
Batch 5 ionophore 0.3 µM.

Each batch is injected IV at J1 into OF1 mice. The mice are sacrificed 1h30 after injection, and livers are recovered. Fluorescence incorporated in the liver macrophages expressing F4/80 marker, the liver cells expressing CD11b marker and the liver dendritic cells expressing the CD11c marker were measures using flow cytometry.

Percentage of liver cells having phagocyted the FITC-dextran containing red blood cells 1h30 after injection into mice (Table 2):

| Bathes | Macrophages F4/80 | CD11b cells | Dendritic cells |
|---|---|---|---|
| 1 | 7.8% | 1.7% | 3.4% |
| 2 | 7.2% | 6.4% | 7.1% |
| 3 | 13.0% | 7.8% | 10.2% |
| 4 | 10.7% | 6.4% | 11.4% |
| 5 | 7.4% | 3.3% | 7.2% |

BS3 and ionophore treatments induce erythrophagocytosis by macrophages (P4/80 and CD11b) and dendritic cells. For BS3 treatment, the percentage of cells that phagocyte treated red blood cells is dose dependant of the amount of BS3 used for treatment.

Example 7. Method for encapsulating ovalbumin in murine and human red blood cells

Variant 1:

Ovalbumin (protein of 45 kDa, hen egg ovalbumin) was encapsulated in murine red blood cells (OF1 mice or C57Bl/6 mice) by the method of hypotonic dialysis in dialysis tubing. The red blood cell suspension was washed several times before being brought to a haematocrit of 70% for the dialysis. The dialysis was carried out in dialysis tubing in a lysis buffer of low osmolarity for about 1 hour or 30 min when the dialysis occured after a heat treatment. The red blood cells were then resealed by means of a solution of high osmolarity for 30 minutes. After a few washes, the final product was taken up in a buffer, Sag-mannitol, and haematocrit was brought to 50%.

Variant 2 :

Ovalbumin was herein encapsulated in the murine red blood cells by the method of hypotonic dialysis in a dialysis column. The red blood cell suspension was washed several times before being brought to a haematocrit of 70% for the dialysis. The dialysis was carried out in a dialysis column in a lysis buffer of low osmolarity for about 10 min. As soon as they left the column, the red blood cells were resealed by means of a solution of high osmolarity for 30 minutes at 37°C. After a few washes, the final product was taken up in a NaCl glucose buffer containing glucose SAG mannitol, or decomplemented plasma, and haematocrit was brought back to 50%.

Example 8: Method for encapsulating ovalbumin in mouse red Mood cells

Ovalbumin (Worthington Biochemical Corporation, Lakewood, NJ) was encapsulated into mouse red blood calls by hypotonic dialyses. Red blood cells suspensions were prepared from C57BL/6 mouse blood collected on lithium heparin. Briefly, the red blood cells were washed three times with saline solution and the haematocrit (Hct) of the blood was adjusted to 70% before dialysis. OVA were added to the red blood cells suspension at a final concentration of 5, or 0.5 mg/ml. Dialysis was performed (cell flow rate of 2ml/min) against a cell lysis buffer (osmolality of 50 mOsmol/kg) circulating at counter-current (15 ml/min) into an 80 hollow-fiber dialyser (Gambro, Lyon, France). Red blood cells were resealed "on-line" by adding (10% final volume) an hypertonic solution (1900 mOsmol/kg) containing 0.4 g/l adenine (Sigma-Aldrich, Saint-Louis, Ml), 15.6 g/l inosine (Sigma-Aldrich), 6.4 g/l sodium pyruvate (Sigma-Aldrich), 4.9 g/l monosodium phosphate dehydrate (Sigma-Aldrich), 10.9 g/l disodium phosphate dodecahydrate (Sigma-Aldrich), 11.5 g/l glucose monohydrate (Sigma-Aldrich) and 50 g/l NaCl (Sigma-Aldrich). Red blood cells were incubated 30 min at 37 °C with the hypertonic solution. Following several washings with 0.9% NaCl 0.2% glucose (Bioluz, Saint-Jean-de-Luz, France), the product was washed once with the tampon A containing Hepes 10 mM, NaCl 140 mM, BSA 0.1%, CaCl2 2.5 mM, diluted with tampon A to $1.10^6$ cells/µl and treated with 0.15µM of Calcium ionophore A23187 (Sigma) for 30 min at 37°C as described in the example 15. After 3 washes with .9% NaCl 0.2% glucose, the final product was resuspended and its haematocrit was adjusted to 50% with decomplemented C57BL/6 mouse plasma (15% final volume). The product thus obtained was stored at 2-8 °C

Example 9: Method for encapsulating ovalbumin in mouse red blood cells

Ovalbumin (Worthington Biochemical Corporation, Lakewood, NJ) was encapsulated into mouse red blood cells by hypotonic dialysis. Red blood cells suspensions were prepared from C57BL/6 mouse blood collected on lithium heparin. Briefly, the red blood cells were washed three times with saline solution and the haematocrit (Hct) of the blood was adjusted to 70% before dialysis. OVA were added to the red blood cells suspension at a final concentration of 5, or 0.5 mg/ml. Dialysis was performed (cell flow rate of 2ml/min) against a cell lysis buffer (osmolality of 50 mOsmol/kg) circulating at counter-current (15 ml/min) into dialysis tubing. After dialysis, red blood cells were resealed by adding (10% final volume) an hypertonic solution (1900 mOsmol/kg) containing 0.4 g/l adenine (Sigma-Aldrich, Saint-Louis, MI), 15.6 g/l inosine (Sigma-Aldrich), 6.4 g/l sodium pyruvate (Sigma-Aldrich), 4.9 g/l monosodium phosphate dehydrate (Sigma-Aldrich), 10.9 g/l disodium phosphate dodecahydrate (Sigma-Aldrich), 11.5 g/l glucose monohydrate (Sigma-Aldrich) and 50 g/l NaCl (Sigma-Aldrich). Red blood cells were incubated 30 min at 37 °C with the hypertonic solution and then chemically treated with BS3 as described in the example 14.
Following several washings with 0.9% NaCl 0.2% glucose (Bioluz, Saint-Jean-de-Luz, France), the final product was resuspended and its haematocrit was adjusted to 50% with decomplemented C57BL/6 mouse plasma (15% final volume). The product thus obtained was stored at 2-8 °C

Example 10. Antibody treatment on the red blood cells containing ovalbumin

The suspension of red blood cells encapsulating ovalbumin is washed several times before being brought to $10^9$ cells/ml for the *in vivo* test and $10^8$ cell/ml for the *in vitro* test. It is incubated with the anti-TER119 antibody (1.0 $\mu$g/ml for the *in vitro* test and 23 $\mu$g/ml or 5 $\mu$g/ml for the *in vivo* test) for 30 minutes at 4°C. After a few washes, the final product is taken up in a buffer with injectable qualities, and haematocrit is brought to 50%.

Example 11. Measurement of the phagocytosis of ovalbumin-containing red blood cells by dendritic cells in vitro

The effect of the antibody treatment on the phagocytosis efficiency of the red blood cells obtained according to example 9, by dendritic cells, is measured *in vitro.* The red blood cells are labelled with a fluorescent label, CFSE (carboxyftuorescein succinimidyl ester), for 20 min at 4°C. CFSE is a non-fluorescent dye which diffuses through the cell membrane. Once inside the cell, the molecule becomes fluorescent subsequent to its cleavage by intracellular esterases.
Dendritic cells are isolated from the spleen of C57BI/6 mice using magnetic beads. These beads carry antibodies which recognize the CD11c marker, thereby making it possible to isolate the CD11c* dendritic cell fraction.
The CFSE-labelled or unlabelled red blood cells are then incubated with the dendritic cells ($10 \times 10^6$ cell/ml) at a ratio of 20:1 in a final volume of 200 $\mu$l/well of round-bottomed 96-well culture plates for 4 hours at 37°C and 5% $CO_2$. After culturing for 4 hours, the red blood cells not ingested by the dendritic cells are lysed with $NH_4Cl$, and several washes are carried out. The capture of the CFSE fluorochrome by the dendritic cells is then measured by flow cytometry (R. Segura et al., J. Immunol, January 2006, 176(1): 441-50).
Three populations of red blood cells were tested:

(A) red blood cells loaded with ovalbumin and not labelled with the CFSE fluorochrome.
(B) red blood cells loaded with ovalbumin and labelled with CFSE.
(C) red blood cells loaded with ovalbumin, treated with the anti-TER 119 antibody and labelled with CFSE.

Results

Table 3: Percentage of dendritic cells having phagocytosed fluorescent red blood cells:

| Red blood cell population | % of dendritic cells |
| --- | --- |
| (A) | 4% |
| (B) | 27% |

(continued)

| Red blood cell population | % of dendritic cells |
| --- | --- |
| (C) | 36% |

The murine red blood cells loaded with ovalbumin and treated with the anti-TER 119 antibody were more efficiently phagocytosed by the.dendritic cells isolated from the spleen than the untreated red blood cells *in vitro,* after 4 hours of coculture. 36% of the dendritic cells phagocytosed the red blood cells carrying the antibody, against only 27% in the absence of antibody.

Example 12. Measurement of the phagocytosis of red blood cells containing ovalbumin, by macrophages and dendritic cells of the spleen and liver *in vivo* on mice

This study is an albgenic study since OF1 mice red blood cells containing ovalbumin are injected to not consanguineous C57BI/6 mice.

Three batches of $74 \times 10^7$ red blood cells, from OF1 mice, loaded with ovalbumin (example 9) treated with the anti-TER 119 antibody (as described in example 10) or not treated are prepared. These batches are divided up in the following way:

Batch 1: no antibody treatment
Batch 2: treated with the anti-TER 119 antibody.

Each batch is labelled with CFSE and injected intravenously into C57BI/6 mice. Three hours after the injection, the blood the spleen and the liver of the mice are taken. The percentage of fluorescent red blood cells circulating in the blood of the mice is measured by flow cytometry. The fluorescence incorporated into the spleen macrophages expressing the F4/80 marker, into the liver macrophages expressing the F4/80 marker and into the spleen dendritic cells expressing the CD11c marker is measured by flow cytometry.

Results

Table 4: Percentage of macrophages or dendritic cells from the spleen, having phagocytosed fluorescent red blood cells 3 hours after injection into the mouse:

| Batches | Macrophages | Dendritic cells |
| --- | --- | --- |
| 1 | 28% | 5% |
| 2 | 81% | 11 22% |

3 hours after injection, the murine red blood cells loaded with ovalbumin and treated with the anti-TER 119 antibody are almost no longer present in the blood of the mouse (1%), whereas there are still untreated, ovalbumin-loaded red blood cells in the brood of the mouse (4.6%).

The red blood cells that have been treated with the anti-TER 119 antibody are phagocytosed by the F4/80 macrophages and CD11c dendritic cells of the spleen.

The red blood cells treated with the anti-TER 119 antibody were more efficiently phagocytosed by the F4/80 macrophages of the spleen than the untreated red blood cells. 81% of the -spleen macrophages phagocytosed the antibody-treated red blood calls, against only 28% in the untreated batch (Table 4).

The antibody-treated red blood cells were also more efficiently phagocytosed by the CD11c dendritic cells from the spleen than the untreated red blood cells. Respectively 22% of dendritic cells phagocytosed the antibody-treated red blood cells against only 5% in the case of the untreated red blood cells (Table 4).

Table 5: Percentage of liver macrophages having phagocytosed fluorescent red blood cells 3 hours after injection into the mouse.

| Batches | Macrophages |
| --- | --- |
| 1 | 24% |

(continued)

| Batches | Macrophages |
|---|---|
| 2 | 50% |

The red blood cells treated with the anti-TER 119 antibody are phagocytosed by the F4/80 macrophages of the liver.

The red blood cells treated with the anti-TER 119 antibody were more efficiently phagocytosed by the F4/80 macrophages of the liver than the untreated red blood cells. 50% of the liver macrophages phagocytosed the antibody-treated red blood cells, against only 24% in the untreated batch (table 5).

In conclusion, the binding of the antibody to the red blood cells allowed efficient targeting of the red blood cells in the spleen and the liver, and a significant increase in the percentage of dendritic cells and of macrophages capable of phagocytizing these red blood cells.

Example 13. Measure of percentage of regulatory T cells and their production of anti-inflammatory interleukin-10 (Il-10) after one injection of Poly(I:C) and antibody-treated or untreated ovalbumin-loaded erythrocytes in mice

The purpose of this study was to measure the percentage of regulatory T cells in C57Bl/6 mice after injection of Poly(I:C) and antibody-treated (anti-TER 119) or untreated ovalbumin-toaded erythrocytes.

Two batches of $30 \times 10^7$ antibody-treated or untreated ovalbumin-loaded erythrocytes from OF1 mice were prepared according to example 8. In this study, an equivalent amount of entrapped ovalbumin was injected free and the negative control was the preservative solution of erythrocytes (NaCl glucosed containing 33% of decomplemented mice plasma). The amount of free or entrapped OVA injected to mice was $2\mu g$. The amount of free Poly(I:C) injected to mice was $25\mu g$.

Batch 1: ovalbumin-loaded erythrocytes and Poly(I:C)

Batch 2: antibody-treated ovalbumin-loaded erythrocytes and Poly(I:C)

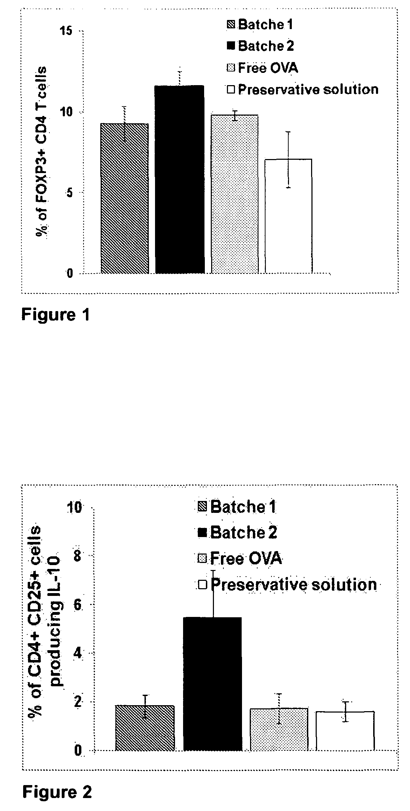

The batches were injected intravenously to C57Bl/6 mice (4 mice per group). Seven days after batch injection, mice were killed and their spleens collected. To measure the percentage of FOXP3 expressing CD4+ T cells by flow cytometry (Figure 1. Table 6), $2.5 \times 10^6$ of spleen cells were used. Briefly, after RBC lysis using $NH_4Cl$ solution (StemCell Technologies, catalogue number 7850). spleen cells were first stained with PC5-anti-CD4 (Biolegend, catalogue number BLE100514) and FITC-anti-CD25 monoclonal antibodies (Biolegend, catalogue number BLE110569), and then incubated with fixation and permeabilisation buffers (Biolegend, catalogue number 421303) before being incubated with PE-anti-FOXP3 mAb (Biolegend, catalogue number 320008) or isotype control.

To measure the percentage of regulatory T cells (CD4+ CD25+) producing IL-10 by flow cytometry (Figure 2), spleen cells ($5 \times 10^6$ cells /ml) were cultured with 0.1 $\mu g/ml$ of OVA323-339 peptide (Genscript, catalogue number 41007-1) for 4 hours at 37°C in 5% $CO_2$-air on 24-well culture plates. One hour after the beginning of the culture, Brefeldin A (Ebioscience, catalogue number 420601) were added to block cytokine secretion. At the end of the culture, cells were first stained with PC5-ariti-CD4 and FITC-anti-CD25 monoclonal antibodies, and then incubated with fixation buffer (Biologend, catalogue number 420801), and permeabilisation buffer (Biolegend, catalogue number 421002) before being incubated with PE-anti-IL-10 mAb (Biolegend, catalogue number 505008) or isotype control.

The Percentage of regulatory CD4 T cells expressing the transcription factor FOXP3 had significantly increased after injection of Poly(I:C) and antibody-treated OVA-loaded erythrocytes or free OVA compared to control mice injected with the preservative solution (Figure 1. Table 6, student test, $p < 0.007$ and $p < 0.05$ respectively).

Table 6: Percentage of CD4 T cells expressing FOXP3

| ITEMS | % of CD4 T cells expressing FOXP3 ($\pm$ standard deviation) |
|---|---|
| Batch 1 | $9.3 \pm 1.1$ |
| Batch 2 | $11.6 \pm 0.9$ |
| Free OVA and Poly(I:C) | $8.6 \pm 1.5$ |
| preservative solution | $7.0 \pm 1.7$ |

Nevertheless, only regulatory T cells induced by antibody-treated OVA-loaded erythrocyte and Poly(I:C) injection can produce the anti-inflammatory cytokine (IL-10) after in vitro restimulation with OVA peptide (Figure 2, table 7).

Table 7: Percentage of regulatory CD4+ CD25+ T cells producing IL-10

| ITEMS | % of CD4 CD25 cells producing IL-10 ($\pm$ standard deviation) |
|---|---|
| Batch 1 | 1.8 $\pm$ 0.5 |
| Batch 2 | 5.5 $\pm$ 2 |
| Free OVA and Poly(I:C) | 1.7 $\pm$ 0.6 |
| Preservative solution | 1.6 $\pm$ 0.4 |

In summary, injection of antibody-treated OVA-loaded erythrocyte and Poly(I:C) induced the generation of regulatory T cells able to produce IL~10 after restimulation with antigen.

In Figure 1, the percentage of FOXP3+ CD4+ T cells in the spleen was determined by flow cytometry 7 days after intravenous injection into C57BL/6 mice of antibody-treated (black bars) or untreated (dark grey bars) OVA-loaded erythrocytes and Poly(I:C) or free OVA and Poly(I:C) (light grey bars), or control medium (white bars). The amount of free or entrapped OVA injected to mice was 2 $\mu$g and the amount of and Poly(I:C) was 25$\mu$g.

In figure 2, the production of IL-10 by regulatory CD4+ CD25+ T cells was determined by flow cytometry after *in vitro* restimulation with OVA peptide 0.1 $\mu$g/ml of spleen cells isolated from C57BL/6 mice injected 7 days with antibody-treated (black bars) or untreated (dark grey bars) OVA-loaded erythrocytes and Poly(I:C) or free OVA (light grey bars) and Poly(I:C), or control medium (white bars). The amount of free or entrapped OVA injected to mice was 2 $\mu$g and the amount of and Poly(I:C) was 25$\mu$g.

Example 14. BS3 treatment on the red blood cells containing ovalbumin

The suspension of red blood cells encapsulating OVA (example 8) was washed several times before being brought to $1.7 \times 10^6$ cells/$\mu$l with PBS and mixed with one volume of a buffer solution of 2mM BS3 (the BS3 solution contained glucose 0,09% and phosphate buffer, pH7.4), so as to obtain a final BS3 concentration of 1mM. The cells were incubated for 30 minutes at room temperature. The reaction was quenched by adding one volume of 20mM Tris-HCl, NaCl 140mM. After incubation at room temperature for 5 minutes, the mixture was centrifuged at 800 g for 5 min, 4°C. The cells were then washed thrice with NaCl glucose (centrifugation at 800 g) for 10 min, before adjustment to hematocrit 50% with decomplemented plasma.

Example 15. Ionophore treatment on the red blood cells containing ovalbumin

The suspension of red blood cells encapsulating OVA (example 8) was washed once with a tampon A containing Hepes 10 mM, NaCl 140 mM, BSA 0.1%. CaCl2 2.5 mM, and then the suspension was diluted to $1.10^8$ cells/$\mu$l using tampon A. Ionophore concentrated in DMSO was diluted with tampon A and then added to the cell suspension in order to get a final concentration of 0.15$\mu$M. The cells were incubated 30 min at 37°C. The mixture was centrifugated at 800 g during 6 min, 4°C. The cells were then washed thrice with NaCl glucose (centrifugation at 800 g) for 10 min, before adjustment to hematocrit 50% with decomplemented plasma.

Example 16. Measure of percentage of regulatory T cells in_ the liver and in the spleens after one injection of ovalbumin-loaded erythrocytes in mice treated to target the liver and/or to repress APC proinflammation response

The purpose of this study was to demonstrate that the use of RBC, treated to target the liver and to repress APC proinflammation responses, as antigen delivery system induced an increase in the percentage of regulatory T cells in mice.

Batches of $126 \times 10^7$ antibody-treated, BS3~treated or ionophore-treated ovalbumin-loaded erythrocytes from C57BL/6 mice were prepared according to example 8. The amount of entrapped OVA injected to mice was 8 $\mu$g.

Batch 1: Ionophore-treated ovalbumin-loaded erythrocytes

Batch 2: BS3-wreated ovalbumin-loaded erythrocytes

Batch 3: antibody-treated ovalbumin-loaded erythrocytes

Batch 4: antibody-treated ovalbumin-loaded erythrocytes and Poty(I:C)

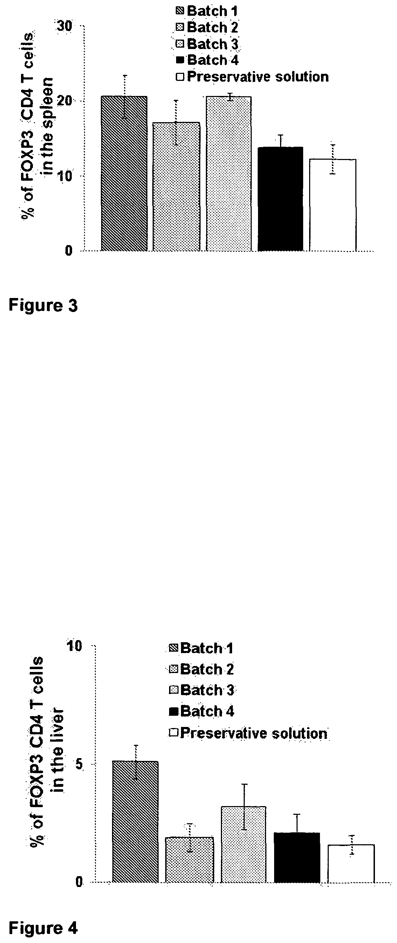

The batches were injected intravenously to C57BI/6 mice (3 mice per group). Seven days after batch injection, mice were killed and their spleens and liver collected. To measure the percentage of FOXP3 expressing CD4+ T cells by flow cytometry in the spleen (Figure 3, Table 8) and in the liver (Figure 4, Table 8), $1 \times 10^6$ and $2.5 \times 10^6$ of liver cells and spleen cells were used. Briefly, after RBC lysis using NH4Cl solution (StemCell Technologies, cat nb 7850), cells were first stained with PC5-anti-CD4 (Biolegend, cat nb BLE100514) and FITC-anti-CD25 monoclonal antibodies (Biologend, cat nb BLE110569), and then incubated with fixation and permeabilisation buffers (Biolegend, cat nb 421303) before being incubated with PE-anti-FOXP3 mAb (Biolegend, cat nb 320008) or isotype control.

In figure 3. the percentage of FOXP3+ CD4+ T cells in the spleen was determined by flow cytometry 7 days after intravenous injection into C57BL/6 mice of ionophore-treated (dark grey bar), BS3-treated (grey bar) or antibody-treated (light grey bar) OVA-loaded erythrocytes or antibody-treated OVA-loaded erythrocytes and Poly(I;C) (black bar) or control medium (white bar). The amount of OVA entrapped injected to mice was $8\mu g$.

In figure 4, the percentage of FOXP3+ CD4+ T cells in the liver was determined by flow cytometry 7 days after intravenous injection into C57BL/6 mice of ionophore-treated (dark grey bar), BS3-treated (grey bar) or antibody-treated (light grey bar) OVA-loaded erythrocytes or antibody-treated OVA-loaded erythrocytes and Poly(I:C) (black bar) or control medium (white bar). The amount of OVA entrapped injected to mice was $8\mu g$.

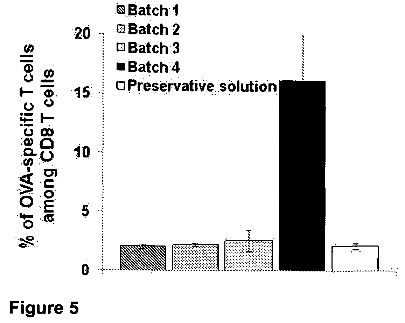

To measure the percentage of OVA-specific CD8 T cells by flow cylometry (Figure 5, Table 9), spleen cells were stained with PC7-anti-CD8 (Biolegend, cat nb BLE100722), FITC-anti-CD3 (Biolegend, cat nb BLE100203) and PC5-anti-CD62L monoclonal antibodies (Biolegend, cat nb BLE104410) and PE-OVA-tetramer (Beckman Coulter, cat nb T20076).

In figure 5, the percentage of OVA-specific CD8+ T cells in the spleen was determined by flow cytometry 7 days after intravenous injection into C57BL/6 mice of ionophore-treated (dark grey bar), BS3-treated (grey bar) or antibody-treated (light grey bar) OVA-loaded erythrocytes or antibody-treated OVA-loaded erythrocytes and Poly(I:C) (black bar) or control medium (white bar). The amount of OVA entrapped injected to mice was $8\mu g$.

To measure the percentage of regulatory T cells (CD4+ CD25+) producing IL-10 by flow cytometry (Table 10), spleen cells ($5 \times 10^6$ cells /ml) were cultured with 0.1 $\mu g$/ml of OVA323-339 peptide (Genscript, cat nub 4100T-1) or without peptide for 4 hours at 37°C in 5% CO2-air on 24-well culture plates. One hour after the beginning of the culture, Brefeldin A (Ebioscience, cat nb 420601) were added to block cytokine secretion. At the end of the culture, cells were first stained with PC5-anti-CD4 and FITC-anti-CD25 monoclonal antibodies, and then incubated with fixation buffer (Biolegend, cat nb 420801), and permeabilisation buffer (Biolegend, cat nb 421002) before being incubated with PE-anti-iL-10 mAb (Biolegend, cat nb 505008) or isotype control.

The ionophore treatment was the only treatment able to induce a significant increase of regulatory CD4 T cells expressing FOXP3 in both the spleen and the liver (Table 8 and, Figures 3 and 4, $p < 0.05$). The antibody treatment was able to induce a significant increase of regulatory CD4 T cells expressing FOXP3 in the spleen only (Table 8, Figure 3, $p < 0.05$).

Table 8: Percentage of regulatory FOXP3 CD4+ T cells in the spleen and the liver

| ITEMS | % of CD4 expressing FOXP3 in the spleen ($\pm$ standard deviation) | % of CD4 expressing FOXP3 in the liver ($\pm$ standard deviation) |
|---|---|---|
| Batch 1 | $20 \pm 3$ | $5 \pm 1$ |
| Batch 2 | $17 \pm 3$ | $2 \pm 1$ |
| Batch 3 | $21 \pm 0$ | $3 \pm 1$ |
| Batch 4 | $14 \pm 2$ | $2 \pm 1$ |
| Preservative solution | $12 \pm 2$ | $2 \pm 0$ |

Only the co-injection of Ab-treated OVA-loaded RBC and Poly(I:C) induced an increase in the percentage of OVA-specific CD8 T cells (Table 9, Figure 5).

Table 9: Percentage of OVA-specific CD8+ T cells in the spleen

| ITEMS | % of OVA-specific CD8 T cells ($\pm$ standard deviation) |
|---|---|
| Batch 1 | $2 \pm 0$ |
| Batch 2 | $2 \pm 0$ |

(continued)

| ITEMS | % of OVA-specific CD8 T cells ($\pm$ standard deviation) |
|---|---|
| Batch 3 | 3 $\pm$ 1 |
| Batch 4 | 16 $\pm$ 7 |
| Preservative solution | 2 $\pm$ 0 |

Only the co-injection of Ab-treated OVA-loaded RBC and Poly(I:C) induced an increase in the percentage of CD4$^+$ CD25$^+$ T producing IL-10 in the spleen and this production was not specific to OVA (Table 10).

Table 10: Percentage of regulatory CD4$^+$ CD25$^+$ T producing IL-10 in the spleen

| ITEMS | Restimulation with OVA peptide ($\pm$ standard deviation) | Restimulation without OVA peptide ($\pm$ standard deviation) |
|---|---|---|
| Batch 1 | 5 $\pm$ 1 | 6 $\pm$ 1 |
| Batch 2 | 4 $\pm$ 0 | 7 $\pm$ 7 |
| Batch 3 | 7 $\pm$ 6 | 9 $\pm$ 1 |
| Batch 4 | 16 $\pm$ 5 | 21 $\pm$ 7 |
| Preservative solution | 4 $\pm$ 1 | 7 $\pm$ 1 |

Example 17. Measure of immune tolerance to OVA after three injections of ovalbumin-loaded erythrocytes in mice treated to target the liver and repress APC proinflammafion response

The purpose of this study was to demonstrate that injections of OVA-loaded RBC, treated to target the liver and repress APC proinflammation response, inhibited the OVA T and B cell responses induced by OVA and Poly(I:C).

Before treatment, samples of blood (200µl) from the C57BL/6 mice were collected by retro-orbital puncture on serum gel separator tube (Becton Dickinson, Microtainer TM SST, ref 365951) to obtain the pre-immun sera. Mice were then injected intravenously thrice at days -7, -3 and -1 with control medium, free OVA or batch of ionophore-treated ovalbumin-loaded erythrocytes prepared with blood from C57BL/6 mice and according to example 8 (3 to 4 mice per group). The amount of free and entrapped OVA injected to mice was 120 and 90 µg, respectively. At day 0 and 21, the mice were challenged with OVA and Poly(I:C) (100µg and 50µg/mice, respectively). Some mice received the control medium only. Six days after the last injection, samples of blood (200µl) from the mice were collected to obtain the post-immun sera and a suspension of CFSE-labelled C57BL/6 splenocytes presenting or not the OVA257-264 peptide at a 1:1 ratio were injected to the mice to evaluate the capacity of cytotoxic CD8 T cells to lyse SIINFEKL cells. 16 hours after the injection of OVA257-264 cells, the mice were killed and their spleens collected.

To measure the percentage of activated and OVA-specific CD8 T cells by flow cytometry (Table 11), spleen cells were stained with PC7-anti-CD8 (Biolegend, cat nb BLE100722), FITC-anti-CD3 (Bialogend, cat nb BLE100203) and PC5-anti-CD62L monoclonal antibodies (Biolegend, cat nb BLE104410) and PE-OVA-tetramer (Beckman Coulter, cat nb T20076).

To measure the percentage of OVA-specific in vivo lysis (Table 12), the percentage of CFSElow and CFSEhigh cells were measured by flow cytometry and determined by the following formula:

$$\% = [1 - (\text{ratio of treated mice /ratio of untreated mice})] \times 100, \text{ with ratio = percentage}$$

$$\text{of CFSEhigh/percentage of CFSElow}$$

To measure the anti-OVA IgG1 and IgG2a titer in the sera (Table 13), various dilutions of the pre- and post-immun sera (from 1/50 to 1/36450) were incubated in a 96-well MaxiSorp plates (Nunc, cat nb 442404) pre-coated with OVA (Serlabo, cat nb WQ-LS003054, 5µg/ml). The presence of anti-OVA IgG1 and IgG2a was revealed by incubation of HorseRadishPeroxidase (HRP)-conjugated to anti-mouse IgG1 (Thermo Scientific, cat nb cat PA1-86031, dilution 1/4000) or anti-mouse IgG2a (Thermo Scientific, cat nb cat PA1-86039, dilution 1/4000) followed by the tetramethylbenzidine (TMB) susbtrat (Biolegend, cat nb 421101) incubation. The reaction was stopped by a solution of 2N H2SO4 and optical density was measured at 450nm and 630nm using a plate reader (Biotek, cat nb ELx808). Data obtained at 630

EP 2 493 487 B1

nm were subtracted to the data obtained at 450 nm and the antibody titer was determined as the dilution for which the optical density is higher than 3 times the O.D. obtained for the pre-immun sera diluted at 1/50.

The injections of ionophore-treated OVA-loaded RBC were able to significantly reduce the proliferation and activation of OVA-specific CD8 T cells induced by OVA and Poly(I:C) compared to the injections of OVA (Table 11; $p < 0.05$).

Table 11: Percentage of activated and OVA-specific CD8$^+$ T cells in the spleen

| Mice treatment | % of OVA-specific CD8 T cells ($\pm$ standard deviation) | % of activated OVA-specific CD8 T cells ($\pm$ standard deviation) |
|---|---|---|
| 3x Batch + 2xOVA and Poly(I :C) | 1.5 $\pm$ 0.1 | 19 $\pm$ 9 |
| 3x OVA + 2xOVA and Poly(I :C) | 2.1 $\pm$ 0.9 | 45 $\pm$ 15 |
| 2 x OVA and Poly(I :C) | 4.8 $\pm$ 1.3 | 76 $\pm$ 7 |
| Preservative solution | 1.1 $\pm$ 0.1 | 6 $\pm$ 3 |

The injections of ionophore-treated OVA-loaded RBC were able to significantly reduce the OVA-specific cellular lysis induced by OVA and Poly(I:C) (Table 12; $p<0.01$).

Table 12: Percentage of OVA-specific *in vivo* lysis

| Mice treatment | % of OVA-specific in vivo lysis ($\pm$ standard deviation) |
|---|---|
| 3x Batch + 2xOVA and Poly(I :C) | 61 $\pm$ 15 |
| 3x OVA + 2xOVA and Poly(I :C) | 73 $\pm$ 19 |
| 2 x OVA and Poly(I :C) | 98 $\pm$ 1 |
| Preservative solution | 1 $\pm$ 2 |

Mice pretreated with ionophore-treated OVA-loaded RBC had very low and/or no anti-OVA IgG1 and IgG2a antibody titers compared to mice pretreated with OVA (Table 13).

Table 13: Anti-OVA IgG1 1 and IgG2a antibody titer in the sera

| Mice treatment | Results for each mice | |
|---|---|---|
| | Anti-OVA IgG1 titer | Anti-OVA IgG2a titer |
| 3x Batch + 2xOVA and Poly(I :C) | 0 | 0 |
| | 900 | 0 |
| | 100 | 0 |
| | 100 | 0 |
| 3x OVA + 2xOVA and Poly(I :C) | 12150 | 2700 |
| | 20250 | 450 |
| | 4050 | 900 |
| | 300 | 0 |
| 2 x OVA and Poly(I :C) | 12150 | 8100 |
| | 1350 | 0 |
| | 150 | 150 |
| Preservative solution | 0 | 0 |
| | 0 | 0 |
| | 0 | 0 |
| | 0 | 0 |

Example 18. Measure of immune tolerance to OVA after injections of various quantities of ovalbumin-loaded erythrocytes in mice

The experiment was performed as described in example 17, except that 2 different batches of ionophore-treated ovalbumin-loaded erythrocytes were prepared with 2 different concentrations of OVA, leading to one batch, batch 1, with 53250 ± 6800 OVA molecules per RBC as in example 17 and another batch, batch 2. with 8250 ± 840 molecules per RBC.

C57BI/6 mice were injected intravenously thrice at days -7, -3 and ~1 with 110µl or 30µl of batch 1. 110µl of batch 2, 110µl of free OVA or 110µl of preservative solution. The amount of OVA and RBC injected to mice are presented in Table 14. At day 0 and day 21, the mice were challenged with OVA and Poly(I:C) (100µg and 50µg/mice, respectively). Six days after the last injection, the mice were killed, the spleen collected and the quantity of IgG1, IgG2b and IgG2c was measured in the sera as described in example 17 using HRP-conjugated to anti-mouse IgG2b (Southern Biotech, 1090-05) and anti-mouse IgG2c (Southem Biotech, 1079-05). To measure IFNϒ production, spleen cells (5 x 10$^6$ cells /ml) were first cultured with 0.1 µg/ml of OVA323-339 peptide (Genscript, cat nub 41007-1) or without peptide for 48 hours at 37°C in 5% $CO_2$ air on 24-well culture plates. Then, IFNϒ was measured in the supernatant by flow cytometry using Cytometric Bead Array (BD Bioscience, 558296 and 558266). To measure the percentage of cytotoxic T-Lymphocyte Antigen 4 (CTLA-4) expressing CD4+ CD25+ T cells by flow cytometry, spleen cells were first stained with PC5-anti-CD4 (Biolegend, cat nb BLE100514) and FITC-anti-CD25 monoclonal antibodies (Biolegend, cat nb BLE110569), and then fixed with PBS 1% paraformaldéhyde (Sigma Aldrich, F1635-25ml) and permeabilzed with saponin 0.3% (Sigma Aldrich, 84510) before being incubated with PE-anti-CTLA-4 mAb (BD Pharminghen. cat nb 553720) or isotype control.

Table 14: Quantities of OVA and RBC injected to mice at each injection

| Mice treatment | OVA (µg/mice) | | | OVA-loaded RBC (X10$^9$ RBC/mice) | | |
|---|---|---|---|---|---|---|
| Injection number | 1 | 2 | 3 | 1 | 2 | 3 |
| Batch 1 High dose (110µl/mice) | 99 | 86 | 86 | 1.7 | 1.5 | 1.5 |
| Batch 1 Low dose (30µl/mice) | 24 | 23 | 23 | 0.4 | 0.4 | 0.4 |
| Batch 2 (110µl/mice) | 11 | 8 | 11 | 1.6 | 1.6 | 1.6 |
| OVA (110µl/mice) | 110 | 110 | 113 | | | |

Mice pretreated with Batch 1 High dose, high quantity of OVA and RBC, had significantly lower anti-OVA IgG1, Ig62b and IgG2c antibody titers than mice pretreated with OVA (Table 15, IgG1: p <0.002, IgG2b and IgG2c: p ≤0.05). Moreover, mice pretreated with Batch 2, same number of RBC but lower dose of OVA (8 to 11µg/mice), had also significantly lower anti-OVA IgG1 and IgG2c antibody titers than mice pretreated with OVA (Table 15, IgG$_1$: p <0.006, and IgG2c: p ≤0.05). However, mice pretreated with Batch 1 Low dose, small quantity of OVA and RBC, had significant antibody titers. Thus, the quantity of OVA and RBC injected per mice plays a key role in immune tolerance induction.

Table 15: Anti-OVA IgG1. IgG2b and IgG2c antibody titer in the sera

| Mice treatment | IgG titer for each mice | | |
|---|---|---|---|
| | Anti-OVA IgG1 titer | Anti-OVA IgG2b titer | Anti-OVA IgG2c titer |
| 3x Batch1 High dose + 2xOVA and Poly(I :C) | 900<br>4050<br>50<br>12150 | 8100<br>450<br>300<br>900 | 2700<br>2700<br>100<br>1350 |
| 3x Batch1 Low dose + 2xOVA and Poly(I:C) | 36450<br>12150<br>12150 | 27.00<br>12150<br>2700 | 24300<br>24300<br>36450 |
| 3x Batch2 + 2xOVA and Poly(I:C) | 0<br>2700<br>8100 | 0<br>24300<br>4050 | 300<br>8100<br>900 |

(continued)

| Mice treatment | IgG titer for each mice | | |
| --- | --- | --- | --- |
| | Anti-OVA IgG1 titer | Anti-OVA IgG2b titer | Anti-OVA IgG2c titer |
| 3x OVA + 2xOVA and Poly(I :C) | 36450<br>36450<br>36450<br>36450 | 36450<br>2700<br>36450<br>36450 | 36450<br>2700<br>36450<br>36450 |
| 2 x OVA and Poly(I:C) | 36450<br>12150<br>1350<br>8100 | 2700<br>8100<br>12150<br>24300 | 4050<br>12150<br>12150<br>36450 |
| Preservative solution | 0<br>0<br>0<br>0 | 0<br>0<br>0<br>0 | 0<br>0<br>0<br>0 |

The challenge OVA and Poly(I:C) induced an increase of IFNϒ production in response to OVA stimulation. This increase was observe in the mice pretreated with free OVA but not with mice pretreated with ionophore-treated OVA-loaded RBC Table 16, ($p < 0.005$).

Table 16: IFNϒ production by splenocytes stimulated with OVA class 2-restricted peptide (average $\pm$ standard devriation)

| Mice treatment | IFNϒ(pg/ml) |
| --- | --- |
| 3x Batch1 High dose + 2xOVA and Poly(I:C) | 43 $\pm$ 6 |
| 3x Batch 1 Low dose + 2xOVA and Poly(I:C) | 38 $\pm$ 18 |
| 3x Batch2 + 2xOVA and Poly(I :C) | 41 $\pm$ 10 |
| 3x OVA + 2xOVA and Poly(I:C) | 82 $\pm$ 9 |
| 2 x OVA and Poly(I:C) | 90 $\pm$ 36 |
| Preservative solution | 47 $\pm$ 25 |

As CTLA-4 is a protein which plays an important regulatory role in immune system by transmission of an inhibitory signal to T cells, its expression was measured on CD4 CD25 regulatory T cells by flow cytometry. Mice pretreated with Batch 1 High dose and Batch 2 had significantly higher percentage of CTLA-4 expression in CD4 CD25 regulatory T cells than mice pretreated with OVA and mice which have received the OVA + Poly(I:C) challenge only (Table 17, Batch 1: p s 0.03 and $p \leq 0.02$, respectively and Batch 2: $p \leq 0.05$). Moreover, mice pretreated with Batch 1 High dose had also significantly higher mean fluorescence intensity (MFI) of CTLA-4 expression in CD4 CD25 regulatory T cells (Table 17, $p \leq 0.03$). Finally, mice pretreated with Batch 1 Low dose had significantly higher percentage of CTLA-4 expression in CD4 CD25 regulatory T cells than mice which have received the OVA + Poly(I:C) challenge only (Table 17, p s 0.04).

Table 17: Percentage and mean fluorescence intensity (MFI) of CTLA-4 in CD4 CD25 regulatory T cells (average $\pm$ standard deviation)

| Mice treatment | % of CTLA-4+ cells in CD4 CD25 T cells | MFI of CTLA-4 in CD4 CD25 T cells |
| --- | --- | --- |
| 3x Batch1 High dose + 2xOVA and Poly(I:C) | 38 $\pm$ 3 | 361 $\pm$ 12 |
| 3x Batch1 Low dose + 2xOVA and Poly(I:C) | 38 $\pm$ 3 | 363 $\pm$ 13 |

(continued)

| Mice treatment | % of CTLA-4+ cells in CD4 CD25 T cells | MFI of CTLA-4 in CD4 CD25 T cells |
|---|---|---|
| 3x Batch2 +2xOVA and Poly(I:C) | 37 ± 2 | 357 ± 10 |
| 3x OVA + 2xOVA and Poly(I:C) | 32 ± 3 | 341 ± 15 |
| 2 x OVA and Poly(I:C) | 31 ± 3 | 341 ± 6 |
| Preservative solution | 34 ± 6 | 348 ± 26 |

In conclusion, treatment with ionophore-treated antigen-loaded RBC allows preventing or reducing antigen-specific T and B cell response in a preventive model. Not only the quantity of antigen, but also the quantity of RBC injected per mice plays a key role in this therapy.

**Claims**

1. Composition comprising red blood cells encapsulating a peptidic or proteic active principle selected from the group consisting of:

   - a therapeutic peptide, polypeptide or protein ,
   - a peptidic or proteic autoantigen,
   - a transplantation peptidic or proteic antigen, and
   - a peptide, polypeptide or protein inducing an allergic reaction,

   for use to inducing, in a host, an immune tolerance to said peptidic or proteic active principle.

2. Composition for the use according to claim 1, wherein the therapeutic peptide, polypeptide or protein is an antibody or a fragment thereof, a clotting factor or a fragment thereof, an enzyme or a fragment thereof or a growth factor or a fragment thereof.

3. Composition for the use according to claim 2, wherein the active principle is an enzyme for use in Enzyme Replacement Therapy (ERT).

4. Composition for the use according to claim 3, wherein the active principle is a lysosomal enzyme.

5. Composition for the use according to claim 4, wherein the lysosomal enzyme is an enzyme for replacement therapy (ERT) in pompe disease (Glycogen storage disease type II), Fabry disease or Mucopolysaccharidoses disorders MPS I.

6. Composition for the use according to claim 5, wherein the lysosomal enzyme is selected from the group consisting of alphaglucosidase enzyme, laronidase and alphagalactosidase A and agalsidase alpha.

7. Composition for the use according to claim 2, wherein the active principle is a clotting factor to treat Haemophilia.

8. Composition for the use according to claim 7, wherein the clotting factor is Factor VII, Factor VIII or Factor IX.

9. Composition for the use according to claim 1, wherein the active principle is a peptidic or proteic autoantigen.

10. Composition for the use according to claim 9, wherein the active principle is against Rheumatoid Arthritis (RA), Multiple Sclerosis (MS), Juvenile diabetes, Uveitis, an inflammatory bowel disease, Crohn's disease, ulcerative colitis, systemic lupus erythematosus, psoriasis, or acquired myasthenia gravis.

11. Composition for the use according to claim 9, wherein the active principle is myelin basic protein against Multiple Sclerosis (MS), the Beta-cell antigen, the pro-insuline, the insuline-like growth factor-2 (IGF2) or mixtures thereof against Juvenile diabetes, the retinal-S antigen against Uveitis, or acetyl choline receptor against acquired myasthenia gravis.

12. Composition for the use according to claim 1, wherein the active principle is a transplantation peptidic or proteic antigen, and the composition of red blood cells containing said antigen is against graft rejection, preferably kidney, heart or liver graft rejection.

13. Composition for the use according to claim 1, wherein the peptide, polypeptide or protein is one inducing an allergic reaction, and is preferably of food origin.

14. Composition for the use according to any one of the preceding claims, wherein the red blood cells (1) contain the active principle and (2) are in the form of an immune complex with an immunoglobulin, preferably an IgG, which recognizes an epitope at the surface of the red blood cells, so as to promote liver targeting.

15. Composition for the use according to Claim 14, wherein the red blood cells form an immune complex with an anti-rhesus or anti-glycophorin A or anti-CR1 antibody.

16. Composition for the use according to any one of the preceding claims, wherein the red blood cells are chemically treated to target the liver.

17. Composition for the use according to Claim 16, wherein the red blood cells are treated with BS3.

**Patentansprüche**

1. Zusammensetzung umfassend rote Blutzellen, die ein peptidisch oder Proteinaktives Prinzip ausgewählt aus der Gruppe bestehend aus:

   - einem therapeutischen Peptid, Polypeptid oder Protein,
   - einem peptidischen oder Protein-Autoantigen,
   - einem peptidischen oder Protein-Transplantationsantigen und
   - einem Peptid, Polypeptid oder Protein, das eine allergische Reaktion induziert, verkapselt, zur Verwendung zum Induzieren einer Immuntoleranz gegenüber dem peptidisch oder Protein-aktiven Prinzip in einem Wirt.

2. Zusammensetzung zur Verwendung gemäß Anspruch 1, wobei das therapeutische Peptid, Polypeptid oder Protein ein Antikörper oder ein Fragment davon, ein Gerinnungsfaktor oder ein Fragment davon, ein Enzym oder ein Fragment davon oder ein Wachstumsfaktor oder ein Fragment davon ist.

3. Zusammensetzung zur Verwendung gemäß Anspruch 2, wobei das aktive Prinzip ein Enzym zur Verwendung in der Enzymersatztherapie (ERT) ist.

4. Zusammensetzung zur Verwendung gemäß Anspruch 3, wobei das aktive Prinzip ein lysosomales Enzym ist.

5. Zusammensetzung zur Verwendung gemäß Anspruch 4, wobei das lysosomale Enzym ein Enzym zur Ersatztherapie (ERT) bei Morbus Pompe (Glycogen-Speicher-Erkrankung Typ II), Morbus Fabry oder Mukopolysaccharidose-Erkrankungen MPS I ist.

6. Zusammensetzung zur Verwendung gemäß Anspruch 5, wobei das lysosomale Enzym ausgewählt ist aus der Gruppe bestehend aus Alphaglucosidase-Enzym, Laronidase und Alphagalactosidase A und Agalsidase alpha.

7. Zusammensetzung zur Verwendung gemäß Anspruch 2, wobei das aktive Prinzip ein Gerinnungsfaktor zur Behandlung von Hämophilie ist.

8. Zusammensetzung zur Verwendung gemäß Anspruch 7, wobei der Gerinnungsfaktor Faktor VII, Faktor VIII oder Faktor IX ist.

9. Zusammensetzung zur Verwendung gemäß Anspruch 1, wobei das aktive Prinzip ein peptidisches oder Protein-Autoantigen ist.

10. Zusammensetzung zur Verwendung gemäß Anspruch 9, wobei das aktive Prinzip gegen rheumatische Arthritis (RA), multiple Sklerose (MS), juvenilen Diabetes, Uveitis, einer entzündlichen Darmerkrankung, Morbus Crohn, Colitis ulcerosa, Lupus erythematodes, Psoriasis oder erworbene Myasthenia gravis ist.

11. Zusammensetzung zur Verwendung gemäß Anspruch 9, wobei das aktive Prinzip basisches Myelinprotein gegen multiple Sklerose (MS), das Betazell-Antigen, Proinsulin, der Insulinähnlichen Wachstumsfaktor 2 (IGF2) oder Mischungen davon gegen juvenilen Diabetes, das Netzhaut-S Antigen gegen Uveitis oder Acetylcholin-Rezeptor gegen erworbene Myasthenia gravis ist.

12. Zusammensetzung zur Verwendung gemäß Anspruch 1, wobei das aktive Prinzip ein peptidisches oder Protein-Transplantationsantigen ist und die Zusammensetzung von roten Blutzellen enthaltend das Antigen gegen Transplantatabstoßung, vorzugsweise Nieren-, Herz- oder Leber-Transplantatabstoßung ist.

13. Zusammensetzung zur Verwendung gemäß Anspruch 1, wobei das Peptid, Polypeptid oder Protein eines ist, das eine allergische Reaktion auslöst und vorzugsweise von Lebensmitteln stammt.

14. Zusammensetzung zur Verwendung gemäß einem der vorangehenden Ansprüche, wobei die roten Blutzellen (1) das aktive Prinzip enthalten und (2) in Form eines Immunkomplexes mit einem Immunglobulin, vorzugsweise IgG, sind, das ein Epitop an der Oberfläche der roten Blutzellen erkennt, um so die Leber-Anvisierung zu fördern.

15. Zusammensetzung zur Verwendung gemäß Anspruch 14, wobei die roten Blutzellen einen Immunkomplex mit einem anti-Rhesus oder anti-Glycophorin A oder anti-CR1 -Antikörper bilden.

16. Zusammensetzung zur Verwendung gemäß einem der vorangehenden Ansprüche, wobei die roten Blutzellen chemisch behandelt sind, um die Leber anzuvisieren.

17. Zusammensetzung zur Verwendung gemäß Anspruch 16, wobei die roten Blutzellen mit BS3 behandelt sind.

**Revendications**

1. Composition comprenant des globules rouges encapsulant un principe actif peptidique ou protéique sélectionné dans le groupe consistant en :

  - un peptide, un polypeptide ou une protéine thérapeutique,
  - un auto-antigène peptidique ou protéique,
  - un antigène peptidique ou protéique de transplantation, et
  - un peptide, un polypeptide ou une protéine induisant une réaction allergique,

destinée à être utilisée pour induire, chez un hôte, une tolérance immunitaire audit principe actif peptidique ou protéique.

2. Composition destinée à être utilisée selon la revendication 1, dans laquelle le peptide, le polypeptide ou la protéine thérapeutique est un anticorps ou un fragment de celui-ci, un facteur de la coagulation ou un fragment de celui-ci, une enzyme ou un fragment de celle-ci ou un facteur de croissance ou un fragment de celui-ci.

3. Composition destinée à être utilisée selon la revendication 2, dans laquelle le principe actif est une enzyme pour une utilisation dans une thérapie enzymatique substitutive (TES).

4. Composition destinée à être utilisée selon la revendication 3, dans laquelle le principe actif est une enzyme lysosomale.

5. Composition destinée à être utilisée selon la revendication 4, dans laquelle l'enzyme lysosomale est une enzyme pour une thérapie substitutive (TES) dans la maladie de Pompe (maladie de stockage du glycogène de type II), la maladie de Fabry ou des troubles de mucopolysaccharidoses MPS I.

6. Composition destinée à être utilisée selon la revendication 5, dans laquelle l'enzyme lysosomale est sélectionnée dans le groupe consistant en l'enzyme alphaglucosidase, la laronidase et l'alpha-galactosidase A et l'agalsidase alpha.

**7.** Composition destinée à être utilisée selon la revendication 2, dans laquelle le principe actif est un facteur de la coagulation pour traiter l'hémophilie.

**8.** Composition destinée à être utilisée selon la revendication 7, dans laquelle le facteur de la coagulation est le facteur VII, le facteur VIII ou le facteur IX.

**9.** Composition destinée à être utilisée selon la revendication 1, dans laquelle le principe actif est un auto-antigène peptidique ou protéique.

**10.** Composition destinée à être utilisée selon la revendication 9, dans laquelle le principe actif est contre la polyarthrite rhumatoïde (PR), la sclérose en plaques (SEP), le diabète juvénile, l'uvéite, une maladie intestinale inflammatoire, la maladie de Crohn, la colite ulcéreuse, le lupus érythémateux systémique, le psoriasis, ou la myasthénie grave acquise.

**11.** Composition destinée à être utilisée selon la revendication 9, dans laquelle le principe actif est la protéine basique de la myéline contre la sclérose en plaques (SEP), l'antigène des cellules bêta, la proinsuline, le facteur de croissance analogue à l'insuline de type 2 (IGF2) ou des mélanges de ceux-ci contre le diabète juvénile, l'antigène S rétinien contre l'uvéite, ou le récepteur de l'acétylcholine contre la myasthénie grave acquise.

**12.** Composition destinée à être utilisée selon la revendication 1, dans laquelle le principe actif est un antigène peptidique ou protéique de transplantation, et la composition de globules rouges contenant ledit antigène est contre le rejet de greffe, de préférence le rejet de greffe rénale, cardiaque ou hépatique.

**13.** Composition destinée à être utilisée selon la revendication 1, dans laquelle le peptide, le polypeptide ou la protéine est du type induisant une réaction allergique, et est de préférence d'origine alimentaire.

**14.** Composition destinée à être utilisée selon l'une quelconque des revendications précédentes, dans laquelle les globules rouges (1) contiennent le principe actif et (2) sont sous la forme d'un complexe immunitaire avec une immunoglobuline, de préférence une IgG, qui reconnaît un épitope à la surface des globules rouges, de sorte à favoriser un ciblage du foie.

**15.** Composition destinée à être utilisée selon la revendication 14, dans laquelle les globules rouges forment un complexe immun avec un anticorps anti-rhésus ou anti-glycophorine A ou anti-CR1.

**16.** Composition destinée à être utilisée selon l'une quelconque des revendications précédentes, dans laquelle les globules rouges sont chimiquement traités pour cibler le foie.

**17.** Composition destinée à être utilisée selon la revendication 16, dans laquelle les globules rouges sont traités avec du BS3.

**Figure 1**

**Figure 2**

**Figure 3**

**Figure 4**

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 101341 A
- EP 679101 A
- WO 2006016247 A

**Non-patent literature cited in the description**

- **E. BREOUS et al.** *Hepatology,* August 2009, 612-621
- **C. JU et al.** *Chem. Res. Toxicol.,* 2003, vol. 16, 1514-1519
- **A.H. LAU et al.** *Gut,* 2003, vol. 52, 1075-1078
- **ROMERO P.J. ; ROMERO E.A.** *Blood Cells Mol. Dis.,* 1999, vol. 25, 9-19
- **BRATOSIN D. et al.** *Cell Death Differ.,* 2001, vol. 8, 1143-1156
- **R. SEGURA et al.** *J. Immunol,* January 2006, vol. 176 (1), 441-50